# EUROPÄISCHE PATENTANMELDUNG

(11) **EP 2 052 702 A1**
(43) Veröffentlichungstag der Anmeldung: **29.04.2009**
(21) Anmeldenummer: 08105629.3
(22) Anmeldetag: 22.10.2008
(51) Int. Cl.: A61F 13/00

(54) **Körper zum Spreizen von Körperabschnitten und Aufnehmen von Ausscheidungen**

(30) Priorität: 22.10.2007 DE 102007050750
(71) Anmelder: Schlote, Manfred F., 40479 Düsseldorf (DE)
(72) Erfinder: Schlote, Manfred F., 40479 Düsseldorf (DE)
(74) Vertreter: Herzog, Martin

(57) **Zusammenfassung**

Die vorliegende Erfindung betrifft einen Körper (1) zum Spreizen von Körperabschnitten und Aufnehmen von Ausscheidungen, mit einer im Wesentlichen länglichen Grundform, einem Kern (7) und einer Hülle (6), wobei der Kern (7) zumindest aus einem Fasermaterial und die Hülle (6) aus einem Garn (11) oder Gewebe (12) gebildet ist.

## Beschreibung

Die vorliegende Erfindung betrifft einen Körper zum Spreizen von Körperabschnitten und Aufnehmen von Ausscheidungen. Die Erfindung findet vor allem Anwendung im Bereich der täglichen und persönlichen Hygiene von Personen, insbesondere im Rahmen der Behandlung von Erkrankungen.

Im Stand der Technik sind Tampons in verschiedensten Formen bekannt. So kommen diese beispielsweise aus Zellstoff gefertigten Tampons in der Zahnmedizin zum Einsatz. Sie werden dort zur Aufnahme von Speichel und zum Spreizen, beispielsweise des Zahnfleisches bzw. der Wangen, verwendet. Weiterhin sind Tampons als Hygieneartikel für Frauen bekannt, die beispielsweise zur Aufnahme von Regelblutungen bestimmt sind.

Während die bekannten Tampons in ihren jeweiligen Einsatzbereichen die ihnen zugedachte Funktion in zufriedenstellender Weise erfüllen, existieren zur Anwendung im Bereich des menschlichen Anus keine zufriedenstellenden Lösungen. Insbesondere Patienten, welche unter Hämorrhoiden oder Thrombosen der Darmvenen leiden, finden bei den bekannten Ausführungsformen keine zufriedenstellenden Lösungen. Die bekannten Ausführungsformen sind entweder von ihrer geometrischen Grundform her nicht zur Anordnung im Bereich des Anus geeignet oder weisen nicht die erforderliche Beschaffenheit und die damit verbundenen Eigenschaften auf, um einen ausreichenden Tragekomfort bei gleichzeitiger Wahrung der Funktionalität zu gewährleisten.

Aufgabe der vorliegenden Erfindung ist es daher, die sich aus dem Stand der Technik ergebenden Probleme zumindest teilweise zu lösen und insbesondere einen Körper zum Spreizen von Körperabschnitten und Aufnehmen von Ausscheidungen anzugeben, der bei den genannten Personengruppen zu einer dauerhaften Linderung und Heilung der Beschwerden bei gleichzeitig sehr hohem Tragekomfort führt.

Diese Aufgabe wird mit einem Körper gemäß den Merkmalen des Patentanspruchs 1 gelöst. Weitere vorteilhafte Ausgestaltungen der Erfindung sind in den abhängig formulierten Patentansprüchen angegeben. Es ist darauf hinzuweisen, dass die in den abhängig formulierten Patentansprüchen einzeln aufgeführten Merkmale in beliebiger, technologisch sinnvoller Weise, miteinander kombiniert werden können und weitere Ausgestaltungen der Erfindung definieren. Darüber hinaus werden die in den Patentansprüchen angegebenen Merkmale in der Beschreibung näher präzisiert und erläutert, wobei weitere bevorzugte Ausführungsbeispiele der Erfindung dargestellt werden. Vorliegend wird die Aufgabe durch einen Körper zum Spreizen von Körperabschnitten und Aufnehmen von Ausscheidungen gelöst, der eine im Wesentlichen längliche Grundform aufweist und einen Kern sowie eine Hülle umfasst, wobei der Kern zumindest aus einem Fasermaterial und die Hülle aus einem Garn oder Gewebe gebildet ist. Durch die längliche Grundform wird zum Einen die Platzierung des Körpers unmittelbar an der Außenseite des Anus begünstigt. Darüber hinaus kann der Kern durch das Fasermaterial besonders weich und saugfähig gestaltet werden. Die darüber angebrachte Hülle verleiht dem Körper schließlich die notwendige Resistenz gegenüber übermäßigen Aufquellungen bei Kontakt mit Feuchtigkeit. Somit wird ein Körper geschaffen, der auch über längere Zeit von einer Person getragen werden kann und einen besonders komfortablen Sitz aufweist. Die an den Anus anschließenden Gesäßwangen können als Körperabschnitte gespreizt werden, ohne dass Einbussen beim Tragekomfort entstehen. So kann eine besonders gute Belüftung des Anus erreicht werden, was die Behandlung von Hämorrhoiden und Darmvenenerkrankungen unterstützt.

Ganz besonders vorteilhaft ist es dabei, wenn das Fasermaterial oder die Hülle zumindest teilweise aus Baumwolle besteht. Dieses Naturprodukt ist sehr hautverträglich und saugfähig. Besonders bewährt hat es sich dabei, wenn die Baumwolle wenigstens entfettet, gereinigt oder ungedreht ist. Durch das Entfetten und Reinigen der Baumwolle, wie beispielsweise einer Makobaumwolle, kann ein besonders viel Luft umschließender Kern geschaffen werden, der dadurch sehr weich und gut belüftend ist. Ist dazu die Baumwolle noch ungedreht, d. h. haben die Fasern keinerlei Verdrehung zueinander erfahren, sondern sind höchstens ineinander verschränkt, wird dieser Effekt noch begünstigt.

Für den Tragekomfort ist es darüber hinaus auch vorteilhaft, wenn die Hülle an ihren Enden und Kanten versiegelt ist. Hierdurch wird ein unbeabsichtigtes Austreten des Kerns aus der verwendeten Hülle vermieden.

Bei einer ganz besonders bevorzugten Ausführungsform der Erfindung ist darüber hinaus vorgesehen, dass die Hülle wenigstens an den Längsseiten nahtlos ist und nur an den Enden versiegelt ist. Durch den Verzicht auf Längsnähte an den Längsseiten des Körpers wird dieser nahtlos und vermeidet somit unangenehmes Scheuern an den Nahtstellen. Insbesondere an den Endbereichen können die Nähte der Versiegelung so angeordnet werden, dass sie mit dem Körper praktisch nicht in Kontakt kommen. Insbesondere ist dies an den äußersten Enden des länglichen Körpers der Fall. So kann die Hülle beispielsweise aus einem endlos gefertigten Strumpf bestehen, der nach dem Befüllen mit dem Kern an den Enden zugenäht und/oder verklebt und somit versiegelt wird. Das Versiegeln kann beispielsweise durch Kleben, Nähen oder Klammern geschehen.

Für die Anwendung im Bereich des menschlichen Anus hat es sich darüber hinaus als vorteilhaft erwiesen, wenn die Länge des Körpers zwischen 80 und 150 mm beträgt. Diese Längen sind einerseits bei den anatomischen Gegebenheiten bequem zu tragen und weisen andererseits eine ausreichend hohe Saugfähigkeit und Spreizwirkung auf.

Entsprechend hat es sich auch als günstig erwiesen, wenn der Körper einen konstanten oder variierenden Durchmesser im Bereich von 8 bis 30 mm aufweist. Je nach Präferenz und Anatomie der tragenden Person kann so ein Körper mit konstanten oder variierenden Durchmessern geschaffen werden. Auch ist eine Anpassung des Durchmessers an die gewünschten Spreizverhältnisse möglich. Je größer dabei der Durchmesser gewählt wird, desto größer ist auch die mit dem Körper verbundene Spreizwirkung auf die auseinandergehaltenen Körperabschnitte.

Als besonders vorteilhaft hat es sich darüber hinaus auch erwiesen, wenn die Kompressibilität des Körpers größer 60 %, insbesondere größer 75 %, ist. Hierdurch wird erreicht, dass der im Bereich des Anus platzierte Körper eine ausreichend hohe Anpassungsfähigkeit hat, um dort dauerhaft und bequem getragen zu werden.

Um ein komfortables Tragen des Körpers auch über längere Zeit zu gewährleisten ist es darüber hinaus vorteilhaft, wenn wahlweise in der Hülle oder dem Kern ein Zusatzstoff angeordnet ist. Ein solcher Zusatzstoff kann beispielsweise ein Bakterien und sonstige Keime abtötender Wirkstoff sein oder auch ein Deodorant, zur Verbesserung oder Vermeidung von Gerüchen.

Schließlich ist es besonders angenehm für einen Träger, wenn der Körper eine Quellfähigkeit von höchstens 150 %, vorzugsweise von 120% bis 140%, gegenüber einem Ausgangsvolumen aufweist. Im Falle der Kontaktierung des Körpers mit einer Flüssigkeit oder einer Ausscheidung wird somit die maximale Vergrößerung des Körpers auf das 1,5-fache begrenzt, wobei diese einen Maximalwert darstellt. In der Praxis haben sich schon Begrenzungen auf das 1,2 bis 1,4 fache als vorteilhaft, weil komfortabel, bewährt. Dies kann entweder durch eine entsprechende Gestaltung des die Hülle bildenden Gewebes oder des die Hülle bildenden Garns geschehen. Insbesondere eine Kombination von nur geringen oder gar nicht quellfähigen Hüllenwerkstoffen mit quellenden Kernwerkstoffen ist dabei möglich. So kann als Hülle bevorzugt ein nahtloser Strumpf verwendete werden, dessen Dehnfähigkeit so begrenzt ist, dass dieser die Volumenvergrößerung des Körpers begrenzt. Die Quellfähigkeit kann beispielsweise gemessen werden, indem der Körper mittig in eine flache Petrischale gelegt wird und über eine Pipette langsam bei Raumtemperatur von beispielsweise 25°C gereinigtes Wasser (Aqua dest.) zugegeben wird. Die Zugabe dauert solange an, bis der Probekörper kein weiteres Wasser aufnimmt. Danach werden die so bestimmten Volumen des Körpers vor und nach der Zugabe des Wassers in Relation zu einander gesetzt.

Zudem hat es sich für bestimmte Anwendungsfälle als vorteilhaft erwiesen, wenn der Körper einen Härtegrad nach Shore von weniger als 30 und insbesondere von weniger als 20 aufweist. So wurden mit einem Shore-Härte-Prüfgerät der Fa. Zwick, Reutlingen die Shore-Härten bekannter Tampons aus der Frauenhygiene und eines erfindungsgemäßen Körpers gemessen. Dies erfolgte beispielsweise im Rahmen einer Messung der Shore-A-Härte.Während der erfindungsgemäße Körper eine Shore-Härte von 20°-30° aufweist, wurden bei den bekannten Tampons wesentlich größere Härtegrade von 55°-70° gemessen. Durch die erfindungsgemäße Shore-Härtegrade von 30° oder weniger aber wird ein besonders ausgeprägter Tragekomfort erzielt, durch den sich der erfindungsgemäße Körper zusätzlich von den bekannten Tampons unterscheidet.

Die Erfindung sowie das technische Umfeld werden nachfolgend anhand der Figuren näher erläutert. Es ist darauf hinzuweisen, dass die Figuren besonders bevorzugte Ausführungsvarianten der Erfindung zeigen, auf die sie jedoch nicht beschränkt ist. In der Zeichnung zeigt:
Fig. 1: einen erfindungsgemäßen Körper in einer Vorderansicht;
Fig. 2: den erfindungsgemäßen Körper nach Fig. 1 in einer Seitenansicht;
Fig. 3: eine Schnittansicht des Körpers nach Fig. 1; und
Fig. 4: zwei Ausführungsformen der Hülle.

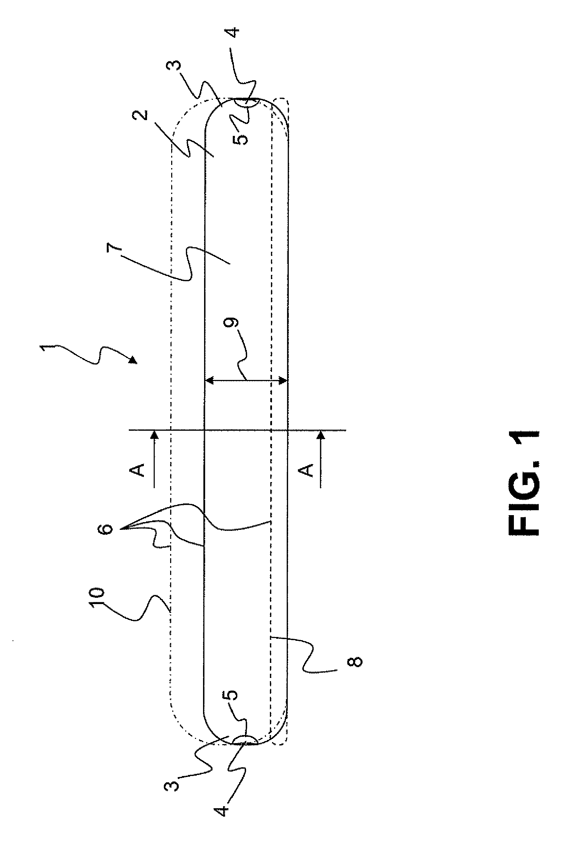

In Fig. 1 ist ein erfindungsgemäßer Körper 1 in einer Vorderansicht dargestellt. Der Körper 1 ist dabei als Baumwoll-Watte-Stab 2 ausgebildet. Der Stab 2 besitzt eine im Wesentlichen längliche Grundform mit zwei gegenüberliegenden Enden 3. An den Enden 3 befindet sich jeweils eine Versiegelung 4, welche als Naht 5 ausgebildet ist. Mit der Naht 5 wird eine Hülle 6, die aus einem endlosen Schlauch gefertigt ist, so verschlossen, dass ein darin eingeschlossener Kern 7 nicht daraus austreten kann.

Weiterhin ist mit einer gestrichelten Linie ein maximaler Kompressionszustand 8 dargestellt. Der Stab 2 kann bei dem dargestellten Ausführungsbeispiel maximal von dem mit der Volllinie gezeigten Normalzustand bis zu diesem Kompressionszustand 8 zusammengedrückt werden. Gegenüber einem ursprünglichen Durchmesser 9 weist der Stab 2 im Kompressionszustand 8 einen Durchmesser auf, der nur ca. 25 % des ursprünglichen Durchmessers 9 entspricht. Damit weist der Stab 2 eine Kompressibilität von 75 % auf.

Weiterhin ist in Fig. 1 mit einer strichpunktierten Linie ein maximaler Quellzustand 10 angedeutet. Der maximale Quellzustand 10 ist dabei so begrenzt, dass er maximal dem 1,5-fachen des ursprünglichen Durchmessers 9 annehmen kann. Das heißt, der Stab 2 wird im Quellzustand 10 an keiner Stelle breiter als der 1,5-fache ursprüngliche Durchmesser 9.

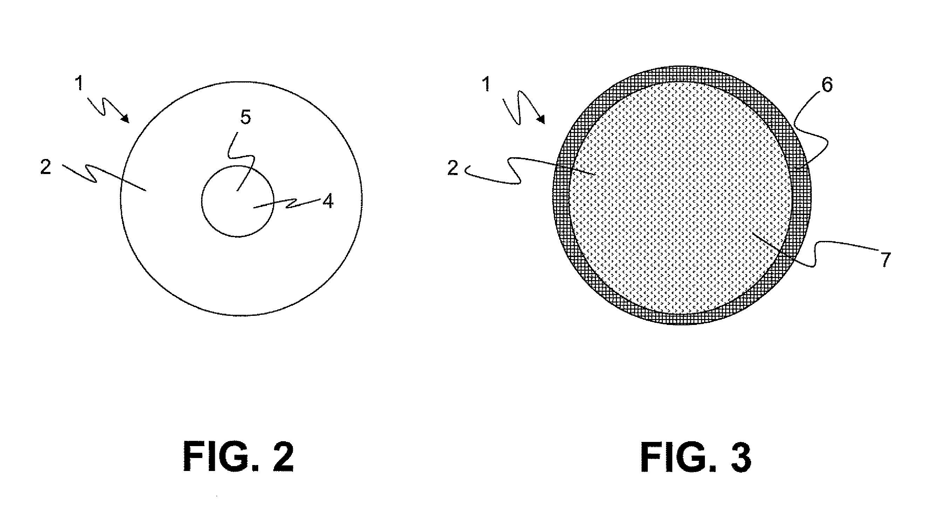

In Fig. 2 ist der Stab 2 nochmals in einer Seitenansicht dargestellt. Gut zu erkennen ist dabei die Lage der Versiegelung 4, welche durch die Naht 5 gebildet ist.

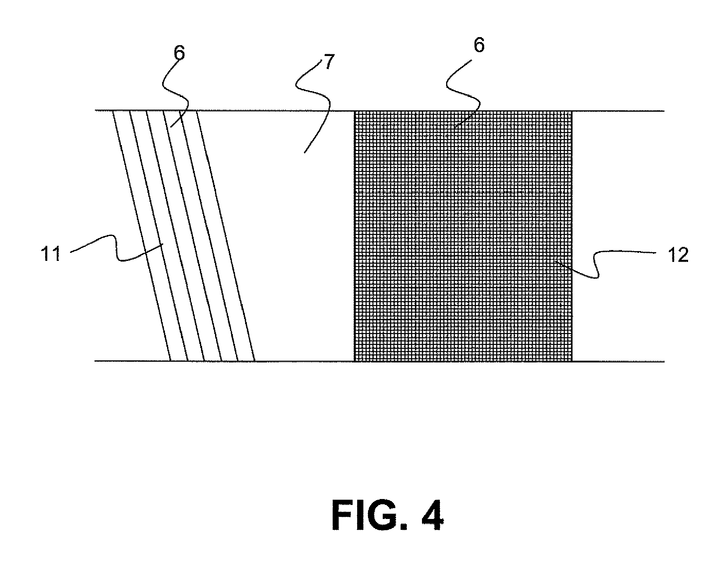

In Fig. 3 ist eine Schnittansicht durch den Körper 1 entlang der Schnittlinie A-A nach Fig. 4 dargestellt. Der Stab 2 weist dabei gut erkennbar eine Hülle 6 auf, welche einen Kern 7 umschließt. Gut erkennbar in dieser Ansicht ist auch, dass die Hülle 6 im Bereich der Längsseite nahtlos ausgebildet ist.

In Fig. 4 sind schließlich zwei mögliche Ausführungsformen für eine erfindungsgemäße Hülle 6 dargestellt. In der linken Hälfte wird die Hülle durch die Umwicklung mit einem Garn 11 erzeugt. Das Garn 11 ist dabei, wie dargestellt, diagonal und spiralförmig um den Kern 7 gewickelt. Bei anderen Weiterbildungen kann auch vorgesehen sein, dass das Garn mehrlagig um den Kern 7 gewickelt ist, d. h. in mehreren Lagen, die sich auch kreuzen können, sogenannten Kreuzwicklungen.

Im rechten Abschnitt der Fig. 4 ist eine weitere Möglichkeit für eine Hülle 6 dargestellt, bei der ein Gewebe 12 verwendet wird. Das Gewebe 12 kann beispielsweise ein Gazestoff sein, wie er aus der Verbandstechnik bekannt ist. Verschiedene mehr oder weniger dünne Fäden werden dazu durch Verweben miteinander zu einem Gewebe verbunden. Durch geeignete Wahl der Quer- und Längsfäden kann dabei in vorteilhafter Weise die Quellfähigkeit und Elastizität des Stabes 2 in der gewünschten Weise eingestellt werden. Gleiches gilt auch für die Verwendung von Fäden für das Garn 11, wobei die Eigenschaften des Garns 11 durch Auswahl der Fäden, der Umwicklungsdichte, Lage und Dehnfähigkeit einstellbar ist.

Mit einem erfindungsgemäß ausgebildeten Baumwollwattestab kann im Bereich des Anus sowohl eine Spreizung als auch eine Aufnahme von möglichen Ausscheidungen sichergestellt werden. Durch die zusätzliche Belüftung und weitestgehende Trockenhaltung des Anus wird die Heilwirkung schließlich in vorteilhafter Weise beeinflusst.

### Bezugszeichenliste

- 1: Körper
- 2: Stab
- 3: Ende
- 4: Versiegelung
- 5: Naht
- 6: Hülle
- 7: Kern
- 8: Kompressionszustand
- 9: Durchmesser
- 10: Quellzustand
- 11: Garn
- 12: Gewebe

## Patentansprüche

1. Körper (1) zum Spreizen von Körperabschnitten und Aufnehmen von Ausscheidungen, mit einer im Wesentlichen länglichen Grundform, einem Kern (7) und einer Hülle (6), wobei der Kern (7) zumindest aus einem Fasermaterial und die Hülle (6) aus einem Garn (11) oder Gewebe (12) gebildet ist.

2. Körper (1) nach Anspruch 1, wobei wenigstens das Fasermaterial oder die Hülle zumindest teilweise aus Baumwolle besteht.

3. Körper (1) nach einem der vorhergehenden Ansprüche, wobei die Baumwolle wenigstens entfettet, gereinigt, oder ungedreht ist.

4. Körper (1) nach einem der vorhergehenden Ansprüche, wobei die Hülle (6) an ihren Enden (3) versiegelt ist.

5. Körper (1) nach einem der vorhergehenden Ansprüche, wobei die Hülle (6) nahtlos in ihrer Längsrichtung ist und nur an den Enden (3) versiegelt ist.

6. Körper (1) nach einem der vorhergehenden Ansprüche, wobei dieser zwischen 80 und 150 mm lang ist.

7. Körper (1) nach einem der vorhergehenden Ansprüche, wobei dieser einen konstanten oder variierenden Durchmesser (9) im Bereich von 8 bis 30 mm aufweist.

8. Körper (1) nach einem der vorhergehenden Ansprüche, wobei die Kompressibilität des Körpers größer 60 %, insbesondere größer 75 % ist.

9. Körper (1) nach einem der vorhergehenden Ansprüche, wobei wahlweise in der Hülle (6) oder dem Kern (7) ein Zusatzstoff angeordnet ist.

10. Körper (1) nach einem der vorhergehenden Ansprüche, wobei dieser eine Quellfähigkeit von maximal 150 %, vorzugsweise von 120% bis 140%, gegenüber einem Ausgangsvolumen aufweist.
